(19) Europäisches Patentamt
European Patent Office
Office européen des brevets

(11) Veröffentlichungsnummer: **0 000 074**
**B1**

(12) **EUROPÄISCHE PATENTSCHRIFT**

(21) Anmeldenummer: **78100147.4**

(22) Anmeldetag: **13.06.78**

(51) Int. Cl.[3]: **C 07 D 471/08,**
A 61 K 31/445,
C 07 D 471/08, 221/00

(54) **Neue Bispidinderivate, Verfahren zu deren Herstellung und Arzneimittel, welche diese enthalten**

(30) Priorität: **13.06.77 DE 2726571**

(43) Veröffentlichungstag der Anmeldung:
**20.12.78 Patentblatt 78/01**

(45) Bekanntmachung des Hinweises auf die Patenterteilung:
**06.08.80 Patentblatt 80/16**

(84) Benannte Vertragsstaaten:
**BE CH DE FR GB NL SE**

(73) Patentinhaber: **BASF Aktiengesellschaft**
**Carl-Bosch-Strasse 38**
**D - 6700 Ludwigshafen (DE)**

(72) Erfinder: **Binnig, Fritz, Dr.**
**Raiffeisenstrasse 31**
**D - 6701 Fussgoenheim (DE)**
**Friedrich, Ludwig, Dr.**
**Nibelungenstrasse 8a**
**D - 6831 Brühl (DE)**
**Hofmann, Hans Peter, Dr.**
**Thorwaldsenstrasse 3**
**D - 6700 Ludwigshafen (DE)**
**Kreiskott, Horst, Dr.**
**Am Boehlig**
**D - 6706 Wachenheim (DE)**
**Mueller, Claus, Dr.**
**Odenwaldring 84**
**D - 6806 Viernheim (DE)**
**Raschack, Manfred, Dr.**
**Donnersbergstrasse 7**
**D - 6714 Weisenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 074 B1

# Neue Bispidinderivate, Verfahren zu deren Herstellung und Arzneimittel, welche diese enthalten

Aus der DT—OS 24 28 792 ist es bereits bekannt, daß bestimmte Bispidinderivate gute Antiarrhythmika sind. Es wurden nun neue Derivate des Bispidins gefunden, die im Vergleich mit bekannten Substanzen eine noch bessere Wirkung besitzen.

Gegenstand der Erfindung sind neue Bispidinderivate der allgemeinen Formel I

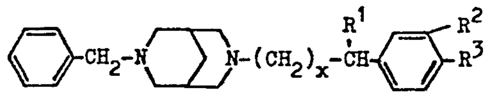

I,

worin

$R^1$ ein Wasserstoffatom oder eine Phenylgruppe,

$R^2$ ein Wasserstoff- oder Chloratom oder eine Trifluormethylgruppe,

$R^3$ ein Wasserstoff-, Fluor- oder Chloratom und

x die Zahl 0 oder 1

bedeuten, wobei jedoch $R^1$, $R^2$ und $R^3$ nicht gleichzeitig Wasserstoffatome sind, sowie deren Salze mit physiologisch verträglichen Säuren.

Gegenstand der Erfindung ist weiter ein Verfahren zu Herstellung von Verbindungen der allgemeinen Formel I und deren Salzenmit physiologisch verträglichen Säuren, welches darin besteht, daß man.

a) N-Monobenzylbispidin mit einer Verbindung der allgemeinen Formel II

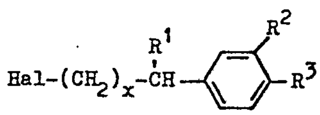

II,

worin $R^1$, $R^2$, $R^3$ und x die obengenannte Bedeutung besitzen und Hal ein Halogenatom ist, umsetzt oder.

b) N-Benzylpiperidon-4 mit Formaldehyd und einem Amin der allgemeinen Formel III

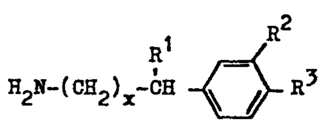

III,

worin $R^1$, $R^2$, $R^3$ und x dasselbe wie oben bedeuten, nach Mannich umsetzt und in dem erhaltenen Bispidon-Derivat die Ketogruppe reduziert und die so erhaltenen Verbindungen gegebenenfalls in ihre Salze mit physiologisch verträglichen Säuren überführt.

Schließlich betrifft die Erfindung auch Arzneimittel, welche Verbindungen der allgemeinen Formel I sowie deren Salze mit physiologisch verträglichen Säuren enthalten.

Als physiologisch verträgliche Säuren kommen z.B. in Frage: Salzsäure, Schwefelsäure, Phosphorsäure, Essigsäure, Malonsäure, Bernsteinsäure, Fumarsäure, Maleinsäure, Zitronensäure, Weinsäure, Milchsäure und Diamidosulfonsäure.

Die Umsetzung des N-Monobenzylbispidins mit den Verbindungen II kann beispielsweise mit Natriumhydrid in Dimethylformamid; Natriumhydroxid in Wasser oder Äthanol; Natriumcarbonat in Butanol oder Amylalkohol; Kaliumcarbonat in Wasser, Methanol, Isopropanol, Butanol, Amylalkohol, Aceton, Acetonitril, Toluol, Dimethylformamid, Dimethylsulfoxid oder Tetrahydrofuran; Natriummethylat in Methanol; Natriumisopropylat in Isopropanol; Kalium-tert.-butylat in tert.-Butanol, Tetrahydrofuran oder Dimethylsulfoxid; Natriumamid in Toluol oder Xylol u.a. durchgeführt werden. Am besten gelingt die Reaktion mit Natriumhydrid in Dimethylformamid. Sie wird in der Regel bei Raumtemperatur durchgeführt.

Die Mannich-Reaktion kann in üblicher Weise vorgenommen werden. Als Lösungsmittel eignen sich z.B. Tetrahydrofuran, Chloroform, Methylenchlorid und besonders gut Methanol, Äthanol und Isopropanol. Als Säuren kommen für die Reaktion vorzugsweise Eisessig und Salzsäure in Frage. Der Formaldehyd kann auch als Paraformaldehyd in die Reaktion eingesetzt werden. Es ist zweckmäßig, die Reaktion bei erhöhten Temperaturen vorzunehmen, wie z.B. bei den Siedepunkten der verwendeten Lösungsmittel. Die Reduktion der so erhaltenen Ketoverbindungen gelingt am besten nach Kishner Wolff.

Die neuen Verbindungen und deren Salze besitzen eine gute antiarrhythmische Wirkung und eine geringe Toxizität.

Zur Bestimmung ihrer antiarrhythmischen Wirksamkeit wurden die Substanzen Ratten (Stamm: Sprague Dawley, Gewicht: 200 bis 250 g) 45 Minuten vor Beginn einer Thiobutabarbital-Narkose (100 mg/kg i.p.) oral appliziert. Als Arrhythmien erzeugende Substanz diente Akonitin, das 60 Minuten nach Substanzapplikation i.v. infundiert wurde (Dosierungsgeschwindigkeit 0,005 mg/kg pro Minute). Bei nicht behandelten Tieren treten nach durchschnittlich 3,32 Minuten Arrhythmien auf, deren Eintritt durch Antiarrhytmica dosisabhängig verzögert werden kann. Die $ED_{50\%}$ ist die Dosis, welche die Infusionsdauer bis zum Auftreten von Arrhythmien um 50% verlängert. R.W. ist die relative Wirkung bezogen auf Chinidin ≡ 1,00. Die maximale Wirkung ist diejenige, die erzielt wird, wenn man die maximal tolerierte Dosis appliziert. Δ% gibt an, um wieviel % die Akonitininfusionsdauer verlängert werden kann. R.M.W. ist die relative maximale Wirksamkeit, bezogen auf Chinidin ≡ 1,00. Die Dosis bei der

toxischen Wirkung gibt die Menge (mg/kg) an, bei der die ersten toxischen Symptome wie Zyanose oder EKG-Veränderungen auftreten. Q ist der Quotient aus toxischer Dosis und $ED_{50\%}$.

Daneben besitzen die neuen Verbindungen und deren Salze calciumantagonistische, antiphologistische und thrombyctenaggregationshemmende Eigenschaften. Sie werden gut resorbiert und sollen oral und parenteral verabfolgt werden. Die täglich Dosis liegt bei etwa 1 bis 20 mg/kg bei oraler Gabe und bei etwa 0,05 bis 1,0 mg/kg bei intravenöser oder intramuskulärer Gabe. Zur Applikation eignen sich z.B. Tabletten, Dragees und Lösungen.

TABELLE

| Substanz | | | | Wirksame Dosis | | Maximale Wirkung | | | Toxische Wirkung | |
|---|---|---|---|---|---|---|---|---|---|---|
| $R^1$ | $R^2$ | $R^3$ | X | $ED_{50\%}$ | R.W. | Dosis | Δ % | R.M.W. | Dosis | Q |
| H | Cl | H | O | 16,6 | 2.57 | 46,4 | 123 | 0,92 | 100 | 6,0 |
| $C_6H_5$ | H | H | O | 20,4 | 2,09 | 215 | 205 | 1,54 | 464 | 22,8 |
| H | H | F | O | 20,2 | 2,11 | 100 | 240 | 1,80 | 215 | 10,6 |
| H | $CF_3$ | H | O | 25,4 | 1,68 | 100 | 242 | 1,82 | 215 | 2,5 |
| H | Cl | Cl | O | 15,6 | 2,74 | 215 | 283 | 2,13 | 464 | 29,7 |
| $C_6H_5$ | H | H | 1 | 13,0 | 3,28 | 46,4 | 203 | 1,53 | 100 | 7,7 |
| Chinidin | | | | 42,7 | 1,000 | 215 | 133 | 1,00 | 464 | 10,9 |

Beispiel 1

a) Herstellung des Ausgangsmaterials

153,2 g (0,5 Mol) N,N'-Dibenzylbispidin (vgl. DT—OS 24 28 792) wurden in 500 ml Äthanol gelöst und nach Zugabe von 5 g 5% Pd/C in einem 2 1-Kolben unter Rühren hydriert. Nach beendeter Wasserstoffaufnahme wurde vom Katalysator abfiltriert, eingedampft und destilliert. Man erhielt 93,2 g (86,3%) N-Monobenzylbispidin, Kp = 98 bis 103°C/0,06 Torr.

b) Herstellung des Endprodukts

Zu einer Lösung von 10,8 g (0,05 Mol) Monobenzylbispidin in 100 ml Dimethylformamid wurden 2,4 g (0,055 Mol) einer 55 %igen Natriumhydridsuspension gegeben. Nach fünfstündigem Rühren bei Raumtemperatur wurden 8,1 g (0,05 Mol) 3-Chlorbenzylchlorid zugetropft und weitere drei Stunden gerührt. Das überschüssige Natriumhydrid wurde mit Methanol zersetzt. Nach dem Abdestillieren der Lösungsmittel im Vakuum wurde der Rückstand in Wasser aufgenommen und mit Äther extrahiert. Nach Trocknen über Natriumsulfat wurde der Äther abgedampft. Der Rückstand wurde in heißem Isopropanol/Essigsäureäthylester gelöst und mit 6 g Fumarsäure versetzt. Beim Abkühlen der Lösung kristalliesierten 18,4 g (= 81,7%) N-Benzyl-N'-(3-chlorbenzyl)-bispidin-fumarat aus, Fp = 181 bis 183°C.

Dasselbe Ergebnis wurde bei Verwendung von 3-Chlorbenzylbromid anstelle von 3-Chlorbenzylchlorid erhalten.

Analog wurden hergestellt:

N - Benzyl - N' - (4 - chlorbenzyl) - bispidin-fumarat,
Fp = 137 bis 139°C, Ausbeute 82,4%

N - Benzyl - N' - (4 - fluorbenzyl) - bispidin-fumarat,
Fp = 170°C, Ausbeute 86,8%

N - Benzyl - N' - (3 - trifluormethylbenzyl)-bispidin-fumarat
Fp = 110°C, Ausbeute 75,5%

N - Benzyl - N' - (3,4 - dichlorbenzyl) - bispidin-fumarat,
Fp = 104°C, Ausbeute 72,1%

N-Benzyl-N'-benzhydryl-bispidin-fumarat,
Fp = 195°C, Ausbeute 86,7%.

Beispiel 2

165 ml Eisessig werden mit 165 ml Methanol vermischt und unter Rühren und Eiskühlung nacheinander 65 g (0,33 Mol) 2,2-Diphenyläthylamin, 62,4 g (0,33 Mol) N-Benzylpiperidon-4 und 24,6 g (0,82 Mol) Paraformaldehyd zugegeben und das Gemisch anschließend 3 Stunden unter Rühren unter Rückfluß gekocht. Nach dem Abkühlen wird der Eisessig und das Methanol in Vakuum bei 50°C weitgehend abgezogen, der Rückstand in 1 000 ml Methylenchlorid aufgenommen und unter Rühren und Eiskühlung mit 20 %iger Natronlauge bis zur stark alkalischen Reaktion versetzt. Die organische Phase wird abgetrennt, die wäßrige Phase mit 200 ml Methylenchlorid extrahiert. Die vereinigten organischen Phasen werden über Natriumsulfat getrocknet und eingedampft. Man erhält 135 g rohes N-Benzyl-N'-2,2-diphenyläthylbispidon. Dieses wird in 500 ml Triäthylenglykol gelöst.

Nach Zugabe von 55 g 85 %igem Kaliumhydroxid werden bei 60 bis 80°C unter Rühren 36,6 g 80 %iges Hydrazinhydrat zugetropft. Danach wird langsam auf 200 bis 210°C erwärmt und 3 Stunden bei dieser Temperatur gehalten, während Wasser über eine 20 cm-Vigreux-Kolonne abdestilliert wird. Nach dem Abkühlen wird das Reaktionsgemisch mit 2 l Wasser verdünnt und 5 × mit je 200 ml Diäthyläther extrahiert. Die vereinigten Extrakte werden über Natriumsulfat getrocknet und eingedampft. Der Rückstand wird im Hochvakuum destilliert, wobei als Hauptfraktion 32,2 g eines zähen gelben Öls vom Kp = 225 bis 250°C/0,12 Torr erhalten wurden. 10 g Fumarsäure werden in wenig siedendem Isopropanol gelöst und mit dem erhaltenen Destillat versetzt. Beim langsamen Abkühlen kristallisiert N - Benzyl - N' - 2,2 - diphenyläthylbispidin-Fumarat × $H_2O$ aus, welches abgesaugt, mit wenig kaltem Isopropanol gewaschen und aus Isopropanol/Wasser umkristallisiert wird. Ausbeute: 17,2 g (= 9,6%), Fp = 142 bis 143°C.

**Patentansprüche**

1. Bispidinderivate der allgemeinen Formel I

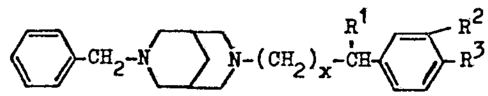

I,

worin

$R^1$ ein Wasserstoffatom oder eine Phenylgruppe,

$R^2$ ein Wasserstoff- oder Chloratom oder eine Trifluormethylgruppe,

$R^3$ ein Wasserstoff-, Fluor- oder Chloratom und

x die Zahl 0 oder 1

bedeuten, wobei jedoch $R^1$, $R^2$ und $R^3$ nicht gleichzeitig Wasserstoffatome sind, sowie deren Salze mit physiologisch verträglichen Säuren.

2. Verfahren zur Herstellung von Bispidinderivaten der allgemeinen Formel I gemäß Anspruch 1, *dadurch gekennzeichnet,* daß man.

a) N-Monobenzylbispidin mit einer Verbindung der allgemeinen Formel II

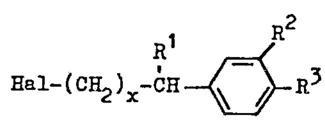

II,

worin $R^1$, $R^2$, $R^3$ and x dasselbe wie in Anspruch 1 bedeuten und Hal ein Halogenatom darstellt, umsetzt oder.

b) N-Benzylpiperidon-4 mit Formaldehyd und einem Amin der allgemeinen Formel III

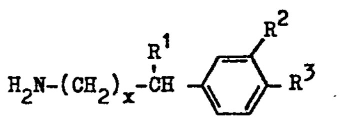

III,

worin $R^1$, $R^2$, $R^3$ und x dasselbe wie in Anspruch 1 bedeuten, nach Mannich umsetzt und in dem erhaltenen Bispidon-Derivat die Ketogruppe reduziert und die so erhaltenen Verbindungen gegebenenfalls in ihre Salze mit physiologisch verträglichen Salzen überführt.

3. Arzneimittel, enthaltend eine Verbindung gemäß Anspruch 1.

4. N - Benzyl - N' - (3,4 - dichlorbenzyl)-bispidin.

5. N-Benzyl-N'-(3-chlorbenzyl)-bispidin.

6. N-Benzyl-N'-(4-fluorbenzyl)-bispidin.

7. N - Benzyl - N' - (3 - trifluormethylbenzyl)-bispidin.

8. N-Benzyl-N'-benzhydryl-bispidin.

9. N - Benzyl - N' - (2,2 - diphenyläthyl)-bispidin.

**Revendications**

1. Dérivés de la bispidine de formule générale I

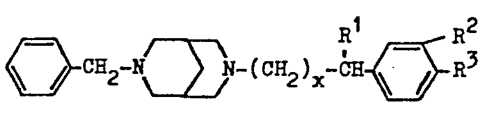

I,

dans laquelle:

$R^1$ est un atome d'hydrogène ou un groupe phényle,

$R^2$ un atome d'hydrogène ou de chlore ou un groupe trifluorométhyle,

$R^3$ un atome d'hydrogène, de fluor ou de chlore et

x le nombre 0 ou le nombre 1,

et les sels de ceux-ci avec des acides tolérés physiologiquement.

2. Procédé de préparation de dérivés de la bispidine de formule générale I selon la revendication 1, caractérise par le fait que:

a) on fait réagir de la N-monobenzylbispidine avec un composé de formule générale II

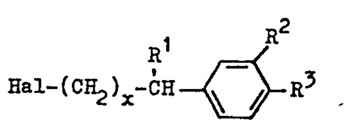

II,

dans laquelle $R^1$, $R^2$, $R^3$ et x ont le même sens que dans la revendication 1 et Hal est un atome d'halogène, ou

b) on fait réagir selon la réaction de Mannich de la N-benzylpipéridone-4 avec du formaldéhyde et une amine de formule générale III

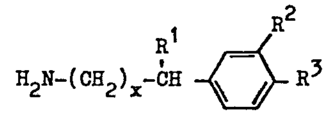

III,

dans laquelle $R^1$, $R^2$, $R^3$ et x ont la même signification que dans la revendication 1, et on réduit le groupe céto du dérivé de bispidone obtenu, et on transforme le cas échéant les composés ainsi obtenus en leurs sels avec des acides tolérés physiologiquement.

3. Médicament contenant un composé selon la revendication 1.

4. N - benzyl - N' - (dichloro - 3,4 benzyl)-bispidine.

5. N-benzyl-N'-(chloro-3 benzyl)-bispidine.

6. N-benzyl-N'-(fluoro-4 benzyl)-bispidine.

7. N - benzyl - N' - (trifluorométhyl - 3 benzyl)bispidine.

8. N-benzyl-N'-benzydryl-bispidine.

9. N - benzyl - N' - (diphényl - 2,2 éthyl)-bispidine.

**Claims**

1. Bispidine derivatives of the general formula I

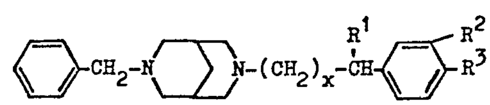

I,

where

$R^1$ denotes a hydrogen atom or a phenyl group,

$R^2$ denotes a hydrogen or chlorine atom or a trifluoromethyl group,

$R^3$ denotes a hydrogen, fluorine or chlorine atom, and

x denotes the number 0 or 1,

and their salts with physiologically acceptable acids.

2. Process for the production of bispidine derivatives of the general formula I according to claim 1, characterized in that

(a) N-monobenzylbispidine is reacted with a compound of the general formula II

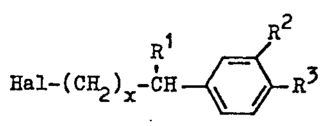

II,

where $R^1$, $R^2$, $R^3$ and x have the same meanings as in claim 1 and Hal denotes a halogen atom, or

(b) N-benzylpiperidone-4 is reacted according to Mannich with formaldehyde and an amine of the general formula III

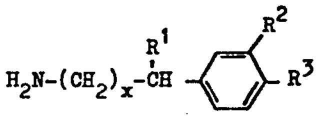

III,

where $R^1$, $R^2$, $R^3$ und x have the same meanings as in claim 1, and the keto group in the bispidone derivative obtained is reduced, and the compounds thus obtained are, if desired, converted into their salts with physiologically acceptable acids.

3. Pharmaceuticals containing a compound as claimed in claim 1.

4. N - Benzyl - N' - (3,4 - dichlorobenzyl)-bispidine.

5. N-Benzyl-N'-(3-chlorobenzyl)-bispidine.

6. N-Benzyl-N'-(4-fluorobenzyl)-bispidine.

7. N - Benzyl - N' - (3 - trifluoromethylbenzyl)-bispidine.

8. N-Benzyl-N'-benzhydryl-bispidine.

9. N-Benzyl-N'-(2,2-diphenylethyl)-bispidine.